(19) Europäisches Patentamt
European Patent Office
Office européen des brevets

(11) EP 2 389 786 B1

(12) **EUROPEAN PATENT SPECIFICATION**

(45) Date of publication and mention
of the grant of the patent:
**11.09.2013 Bulletin 2013/37**

(21) Application number: **10701257.7**

(22) Date of filing: **20.01.2010**

(51) Int Cl.:
*H05B 6/06* *(2006.01)*     *A61N 1/40* *(2006.01)*

(86) International application number:
**PCT/GB2010/000083**

(87) International publication number:
**WO 2010/084311 (29.07.2010 Gazette 2010/30)**

(54) **APPARATUS FOR DRIVING A RESONANT CIRCUIT**

VORRICHTUNG ZUR ANSTEUERUNG EINES RESONANZKREISES

APPAREIL D'EXCITATION D'UN CIRCUIT RÉSONANT

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **21.01.2009 GB 0900993**

(43) Date of publication of application:
**30.11.2011 Bulletin 2011/48**

(73) Proprietor: **UCL Business PLC**
**London W1T 4TP (GB)**

(72) Inventors:
• **HATTERSLEY, Simon, Richard**
**Bickley BR1 2DF (GB)**
• **SOUTHERN, Paul**
**London N7 9SH (GB)**
• **PANKHURST, Quentin, Andrew**
**Hertfordshire AL1 4RJ (GB)**
• **KALLUMADIL, Mathew**
**London NW1 6NG (GB)**

(74) Representative: **Tyson, Robin Edward et al**
**J A Kemp**
**14 South Square**
**Gray's Inn**
**London WC1R 5JJ (GB)**

(56) References cited:
DE-A1-102005 059 751     FR-A1- 2 904 873
US-A- 5 236 410          US-A1- 2001 035 406
US-A1- 2005 067 410

## Description

This invention relates to an apparatus for driving a resonant circuit. For example, the apparatus can be used to provide an alternating magnetic field. The invention also provides a system which comprises a resonant circuit, a sensor and the apparatus for driving the resonant circuit. Applications of the invention include generating an alternating magnetic field to cause magnetic nanoparticles to dissipate heat, particularly for use in magnetic field hyperthermia (MFH).

Heating living tissues between 42°C and 45°C prevents regular intracellular activity and temperatures above that will typically lead to apoptosis or necrosis. A method known as magnetic field hyperthermia utilises the heating properties of magnetic nanoparticles under the influence of an externally applied alternating magnetic field to deliver heat that will lead to hyperthermia. At high alternating magnetic field frequencies typical energy losses occur through Néel relaxation mechanisms rather than Brownian relaxation, which is typically hindered by immobilisation in tissue. Specifically for the range of frequencies used in this system it has been shown that the power dissipation is proportional to frequency and also the square of the field.

The transport of magnetic nanoparticles (MNP) to specific sites can be achieved by a number of mechanisms. In bacterial MFH studies it has been shown that spontaneous take-up of the MNP occurs. Targeting tumours can either be achieved by injecting the MNP suspension directly into the tumour or by labelling the MNP with tumour-specific antibodies.

Further information on magnetic field hyperthermia can be obtained from US 5 236 410, DE 10 2005 059 751 and FR 2 904 873.

Currently, systems used for magnetic hyperthermia generate alternating magnetic fields at frequencies typically around 400kHz, and require large power supplies. For example, a system provided by Magforce (MFH- 300F) , generates magnetic fields with field strengths up to 18kA/m (220 Oe) requiring currents of 500A at 100kHz with a 21- 33cm treatment gap and radius of 10cm. The mobility of the system is severely limited due to size constraints and power requirements. Due to such a large treatment area, local application of magnetic fields to smaller areas is not possible. The large treatment area also limits the strength and frequency of the applied field. Systems for industrial inductive heating such as those provided by MSI Automation and Ameritherm Industrial are 3- phase induction heaters that generate an alternating magnetic field with a frequency between 100- 450kHz. Typical output powers between 1- 5kW are required to transfer enough energy to the work piece to increase temperatures to above 600°C. In contrast, large power outputs are not required for clinical uses of magnetic hyperthermia- a typical clinical hyperthermia experiment requires only a temperature rise of a few degrees, produced by a few watts of output power coupling to the magnetic particles not several kilowatts.

It is an aim of the present invention to alleviate one or more of the above mentioned problems, by presenting a method to produce high intensity, high frequency magnetic fields at low levels of power.

A parallel resonant electric circuit, also known as a tank circuit, is a circuit comprising inductive and capacitive components connected so that it oscillates when subjected to a disturbance. The simplest tank circuit consists of an inductor L and a capacitor C connected in parallel, in which case the natural frequency of oscillation is given by $f = \dfrac{1}{2\pi\sqrt{LC}}$ . This is also the frequency of resonance, the frequency at which a given amplitude of alternating drive current through the tank circuit gives rise to the maximum amplitude of alternating current flow within the tank circuit.

US 2005/0067410, which is considered to represent the closest prior art, discloses an induction heating system having the features of the preamble of claim 1.

The present invention provides an apparatus for driving a resonant circuit, the apparatus comprising: a first drive circuit, the first drive circuit arranged to provide a drive current to said resonant circuit; and a controller coupled to said first drive circuit, the first drive circuit further comprising: a first input adapted to receive a current from a power supply; a switch; a second input adapted to receive from said controller a signal to control said switch; and an output coupled to said resonant circuit; wherein the controller is adapted to receive from a sensor a signal derived from the current flowing in said resonant circuit, wherein said sensor is coupled to said controller, and said controller is configured to close said switch to enable the drive current to flow through said resonant circuit when said signal derived from said sensor satisfies a first condition and said controller is further configured to open said switch to cause the drive current to stop flowing through the resonant circuit when said signal derived from said sensor satisfies a second condition,

characterized by the first drive circuit further comprising: a first inductor, which acts to set the drive current through said resonant circuit when the switch is closed; and a first diode coupled across said first inductor, said first diode arranged to enable current to continue to flow in the first inductor when the switch is open.

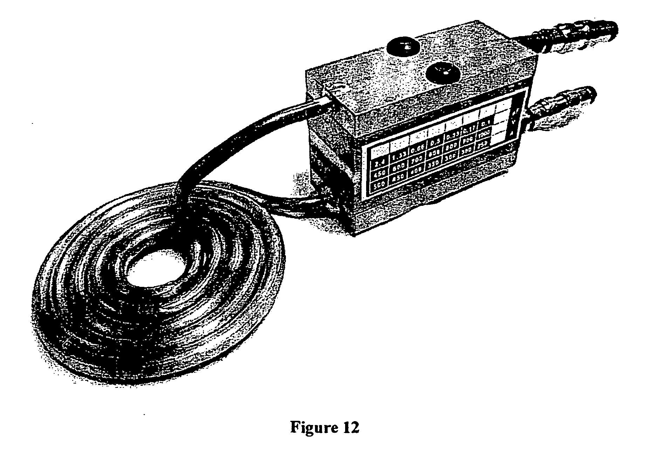

Accordingly, the invention enables the generation of alternating magnetic fields exceeding 16 kA/m (200 Oe) and self- tunes to the resonant frequency of the capacitor/ inductor combination of the resonant circuit at radiofrequencies (at least up to 2 MHz) . The input power requirements are considerably lower than commercially available induction heating systems and therefore would be safer for operation in a clinical environment. Input power requirements are of the order of hundreds of watts rather than several kilowatts as seen in induction heating devices. This is primarily due to the low input requirements to drive a capacitor/ inductor resonant circuit having a high Q- factor. To decrease the resistive losses, and hence raise the Q- factor, it is necessary to use a low- loss high- frequency power capacitor, to make the coil and connections of large surface area because of the skin effect, and to connect the coil (inductor) and capacitor as closely as possible. A low resistance pathway between the coil and capacitor may be achieved by connecting the coil to the capacitor using busbars, or mounting plates as shown in figure 12. The coil is fabricated from copper pipe to allow heat generated from resistive heating to be dissipated from both the capacitor and coil by flowing water or other suitable cooling fluid through the pipe.

In a preferred aspect of the invention, the drive circuit described above further comprises a second diode in series with the above-mentioned switch to substantially stop current flow through said switch in the reverse direction, so as to allow the resonance to be driven to a voltage amplitude exceeding the power supply voltage.

In another preferred aspect of the invention, the drive circuit of any of the above aspects further comprise a second inductor coupled to said resonant circuit to provide smoothing of the current flowing through said resonant circuit.

In another preferred aspect of the invention, an apparatus comprises a plurality of apparatuses according to any one of the preceding aspects.

In another preferred aspect of the invention, the apparatus according to any one of the above aspects further comprise at least one additional drive circuit, wherein the second input of each additional drive circuit is coupled to said controller and the output of each additional drive circuit is coupled to the resonant circuit.

In another preferred aspect of the invention, an apparatus comprises a first and second apparatus according to any one of the above aspects, wherein the polarity of the voltage applied at the first input of the first apparatus is opposite to the polarity of the voltage applied to the first input of the second apparatus. The first and second conditions of said first apparatus are substantially at a phase of 180 degrees relative to the same as the second and first the first and second conditions respectively of the second apparatus. The current flow through the resonant circuit is substantially in one direction when the switch in the first apparatus is closed and substantially in the opposite direction when the switch in the second apparatus is closed.

In another preferred aspect of the invention, the apparatus according to any one of the above aspects further comprise one additional drive circuit, wherein the second input of the additional drive circuit is coupled to said controller and the output of the additional drive circuit is coupled to the resonant circuit. The polarity of the voltage applied at the first input of the first drive circuit is opposite to the polarity of the voltage applied at the first input of the additional drive circuit. The first and second conditions of the first drive circuit are substantially at a phase of 180 degrees relative to the first and second conditions respectively of the additional drive circuit.

The half-bridge configurations of the above aspects allow for higher current in the resonant circuit.

In another preferred aspect of the invention, an apparatus comprises a plurality of apparatuses according to the any one of the above aspects.

In another preferred aspect of the invention, an apparatus comprises a first and second apparatus according to any one of the above aspects, wherein the first drive circuit of the first apparatus is connected to one end of the resonant circuit and the additional drive circuit of the first apparatus is connected to the opposite end of the resonant circuit, and the first drive circuit of the second apparatus is connected to said opposite end of the resonant circuit and the additional drive circuit of the second apparatus is connected to said one end of the resonant circuit. The first and second conditions of the first and additional drive circuit of the first apparatus are substantially the same and first and second conditions of the first and additional drive circuit of the second apparatus are substantially the same and the first and second conditions of said first apparatus are substantially at a phase of 180 degrees relative to the first and second conditions respectively of the second apparatus. The current flow through the resonant circuit is in one direction when the switches in the first apparatus are closed and in the opposite direction when the switches in the second apparatus are closed.

In another preferred aspect of the invention, an apparatus according to any one of the above aspects further comprises a second, third and forth drive circuit, wherein: the second input of the first, second, third and fourth drive circuits are coupled to said controller; the polarity of the voltage applied at the first inputs of the first and fourth drive circuits is opposite to the polarity of the voltage applied at the first inputs of the second and third drive circuits; the outputs of the first and second drive circuits are connected to one end of the resonant circuit and the outputs of the third and fourth drive circuits are connected to the opposite end of the resonant circuit; the first and second conditions of the first and third drive circuits are substantially the same and first and second conditions of the second and fourth drive circuits are substantially the same and the first and second conditions of the first and third drive circuits are substantially at a phase of 180 degrees relative to the first and second conditions respectively of the second and fourth drive circuits. The current flow through the resonant circuit is in one direction when the switches in the first and third drive circuits are closed and in the opposite direction when the switches in the second and fourth drive circuits are closed.

The full-bridge configurations of the above aspects allow for higher current in the resonant circuit than the half-bridge configuration.

In another preferred aspect of the invention, the apparatus in any one of embodiments four to ten have at least two drive circuits connected in parallel. This allows the drive circuits to share the load current.

The invention also provides a system comprising an apparatus according to any one of the above aspects; a resonant circuit comprising a magnetising coil connected to said apparatus; and a sensor adapted to derive a signal related to the current flowing in said resonant circuit.

The modular design of the system allows the user to change the resonant circuit without having to retune or adjust the apparatus, sensor or power supply.

In one aspect of the system, the magnetising coil is interchangeably connectable to said apparatus.

Since the invention requires low power and can adapt to a range of coil geometries, the system does not require specialised power supplies.

In another aspect of the system, the magnetising coil is comprised within a hand-held device.

With the ability to change the geometry of the coil, a highly mobile hand-held device can be used to apply the alternating magnetic field to localised regions where MFH is required.

Embodiments of the invention will now be described, by way of example only, with reference to the accompanying drawings in which:

Figure 1 is a schematic representation of a system including a resonant circuit, a sensor and an apparatus for driving the resonant circuit;

Figure 2 is a schematic representation of the controller of Figure 1;

Figure 3 is a Simulation Program with Integrated Circuit Emphasis (SPICE) circuit diagram of the system with simplified control logic;

Figure 4 is a SPICE simulation of the start-up phase of the circuit shown in Figure 3;

Figure 5 is a SPICE simulation of the stable phase of the circuit shown in Figure 3.

Figure 6 is a schematic diagram of a half-bridge configuration;

Figure 7 is a SPICE model simulation of the configuration shown in Figure 6;

Figure 8 is a schematic diagram of a full-bridge configuration;

Figure 9 is a SPICE model simulation of the configuration shown in Figure 8;

Figure 10 is a schematic diagram of a load-sharing configuration;

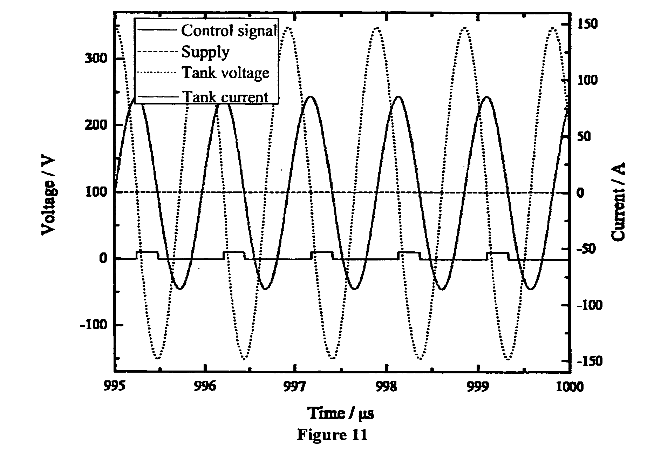

Figure 11 is a SPICE model simulation of the configuration shown in Figure 10.

Figure 12 is a photograph showing a prototype of a hand-held resonant circuit;

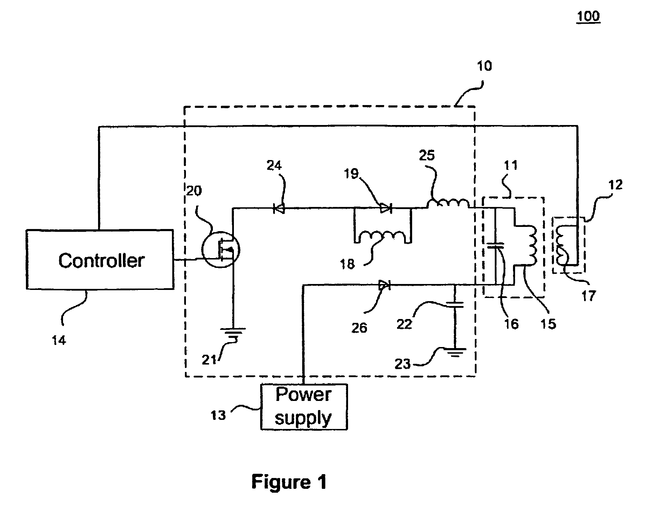

Figure 1 shows a system 100 comprising an electronic circuit 10, a resonant circuit 11, a sensor 12, a power supply 13 and a controller 14. The electronic circuit 10 drives the resonant circuit 11 at a frequency substantially equal to the resonant frequency of the circuit.

The resonant circuit 11 of this embodiment comprises a coil 15 and a capacitor 16 connected in parallel. The resonant circuit 11 may also contain any combination circuits and/or components that are electrically equivalent to a parallel LC circuit that may include inductive and capacitive components and/or circuits which, when connected together, can enable an electrical current to alternate between the inductive and capacitive parts at their resonant frequency.

The sensor 12 is connected to the controller 14 to provide the controller 14 with an electrical signal that is related to the current through the resonant circuit 11. The sensor 12 in this embodiment comprises a sense coil 17 that is inductively coupled to the coil 15 of the resonant circuit 11. When an alternating current flows through the coil 15, an alternating magnetic field is generated, this induces a voltage in the sense coil 17, which provides an electrical signal. Thus sense coil 17 provides the controller 14 with an electrical signal that is related to the current in the coil 15 of the resonant circuit 11. This provides a cleaner, lower voltage feedback signal to the controller 14, compared to using a simple connection from the coil 15 to the controller 14. Alternatively, the sensor 12 may comprise any components that are able to sense an electrical or magnetic signal, such as Hall effect sensors, etc.

The power supply 13 is preferably a high voltage DC source capable of providing, for example, 150 V. The magnetic field in the coil is proportional to the current in the coil, which in turn is proportional to the AC voltage thus proportional to the DC supply voltage. Varying the DC supply voltage from said power supply can therefore control the magnetic field strength.

The electronic circuit 10 of this embodiment comprises an inductor 18 (e.g. a 220 $\mu$H inductor), a reverse diode 19 and a switch 20. Optionally, the electronic circuit 10 may further comprise any one of, or a combination of, a capacitor 22 (e.g., a 2$\mu$F capacitor), a first forward diode 24, a smoothing inductor 25 (e.g. a 2 $\mu$H inductor) or a second forward diode 26. These components must be rated to cope with high voltages and high currents typically up to 600 V and 20 A.

The switch 20 of this embodiment is an n-channel enhancement-mode metal-oxide-semiconductor field-effect transistor (MOSFET). Devices of this type are available to handle high currents, up to 20 A, and high voltages, up to 1000 V, at switching speeds in the range 5 to 50 ns. An insulated-gate bipolar transistor (IGBT), for example, could replace said switch in lower frequency applications.

The power supply 13 is connected to the electronic circuit 10 which controls the flow of current in the resonant circuit 11. The controller 14 applies electrical signals to the switch 20 to open and close the switch 20. When the switch 20 is closed, a current flows from the power supply 13 (or preferably mainly from reservoir capacitor 22) through the resonant circuit 11, through the inductor 18 and through the switch 20 to ground 21.

On application of power the resonant circuit 11 is in a quiescent state until the controller 14 gives it an initial pulse by closing the switch 20 for a short period. This draws current through the coil 15, the rate of build-up of current being set by the much larger inductance of inductor 18. At the end of the initial pulse, when the controller 14 opens the switch 20, the current flow in the coil 15 persists so that it charges power capacitor 16, thus starting the oscillatory exchange of energy between the inductive and capacitive components of resonant circuit 11 at its natural frequency. At the same time, the current through inductor 18 is maintained by flowing around through diode 19.

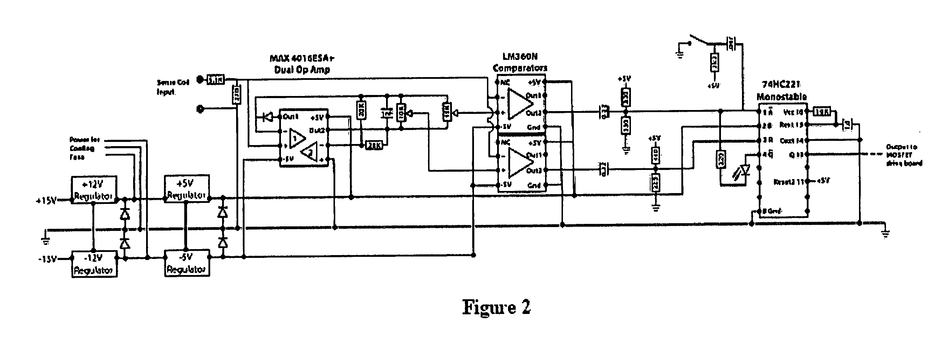

The controller 14, as shown in figure 2, now receives a signal from the sensor 12, from which it can determine the timing of the magnetic field in the coil 15, and starts to operate the switch 20 in step with this signal. Using potentiometers, a relative voltage determined by the detected amplitude from the sensor 12 is fed into comparators to set the phase points. Each time the switch 20 is closed, some additional charge flows out of capacitor 16, increasing the voltage amplitude; each time the switch 20 is opened, the extra voltage causes the current flow in coil 15 to increase slightly. At the same time, the current in inductor 18 progressively increases, so gradually larger pulse of current are drawn through the resonant circuit 11, helping to build the amplitude of resonance. The amplitude continues to grow until resistive heating losses, primarily within the resonant circuit 11 due to the very large resonant current, equal the power supplied by the pulsed current.

If the switch 20 is able conduct in the reverse direction (this is true of MOSFET switches) then it is not possible for the voltage on the resonant circuit 11 to swing much below zero volts. In a preferred embodiment of the apparatus 100, diode 24 restricts the current flow to the direction which increases the amplitude of resonance. This allows the voltage amplitude to significantly exceed the power supply voltage, and the resonant current and magnetic field to be proportionally higher.

The electronic circuit 10 may optionally comprise a smoothing inductor 25 connected in series with the resonant circuit 11. The smoothing inductor 25 provides smoothing of the sinusoidal current flowing through the resonant circuit 11 and is of benefit to provide stable lock-in at the resonant frequency.

The electronic circuit 10 may optionally comprise a capacitor 22, depending upon current capabilities of power supply 13, which is connected between ground 23 and the electrical connection between the power supply 13 and the resonant circuit 11 such that any sudden demand of current may be provided by said capacitor 22.

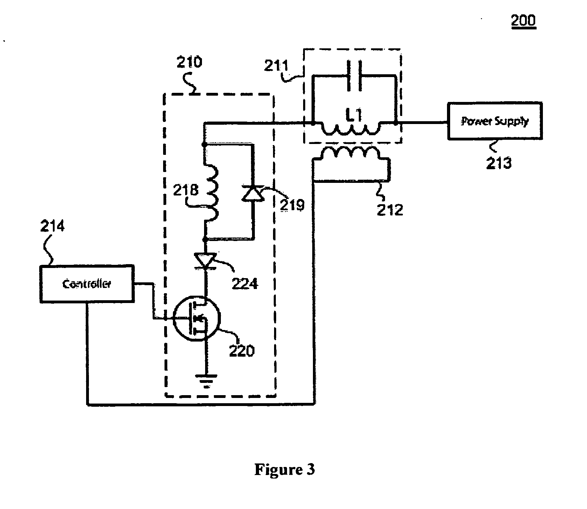

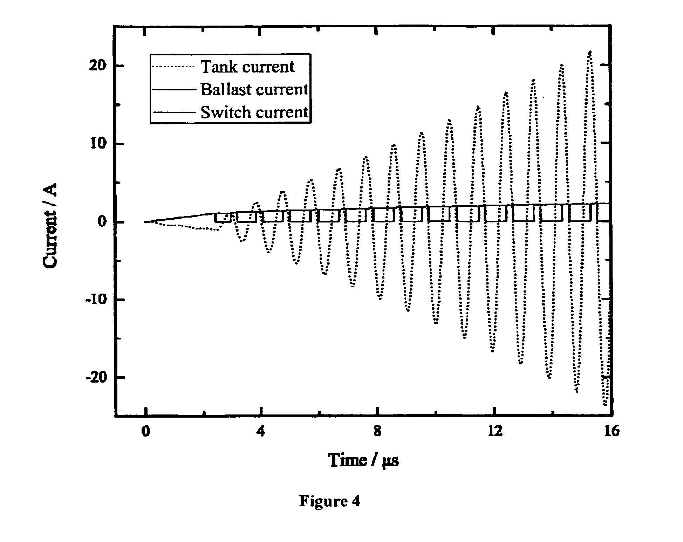

Figure 3 shows a simplified diagram of the system, in which the controller 214 and switch 220 are represented by behavioural models. The controller 214 provides an initial fixed pulse, followed by pulses in step with the oscillation in the resonant circuit 211. Figure 4 shows a simulation of start-up behaviour, showing gradual growth in the current in the inductor 215 and hence in the current pulses through the switch 220, as the resonance current amplitude increases. When providing the pulse, the controller 214 can close the switch 220 at any time from the positive peak voltage in the resonant circuit 211 to zero volts. The controller 214 can then open the switch at any time when the voltage in the resonant circuit 211 is negative. The switch 220 can only be opened after it is closed. Preferably, if phase is taken to equal zero when the voltage in the resonant circuit is at its positive peak, then switch 220 should be closed when the phase is between 50° and 90° and then opened when the phase is between 160° and 200°.

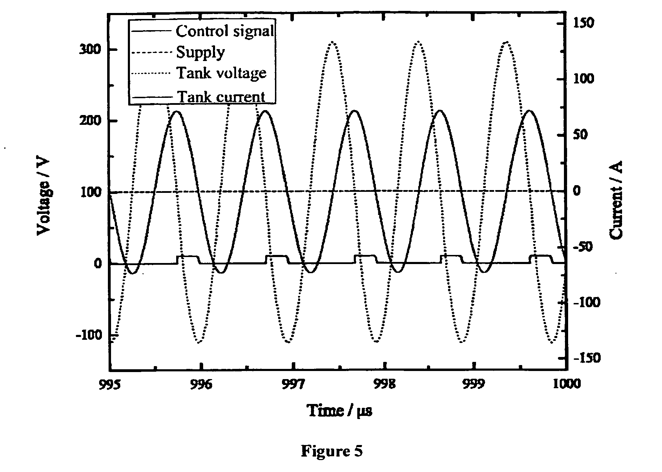

Figure 5 shows stable operation reached after 1 ms, with a peak-peak voltage swing of 400 V, which greatly exceeds the power supply voltage of 100 V. The peak current in the resonant circuit 211 is 68 A, whereas the current in the switch 220 is only 17 A.

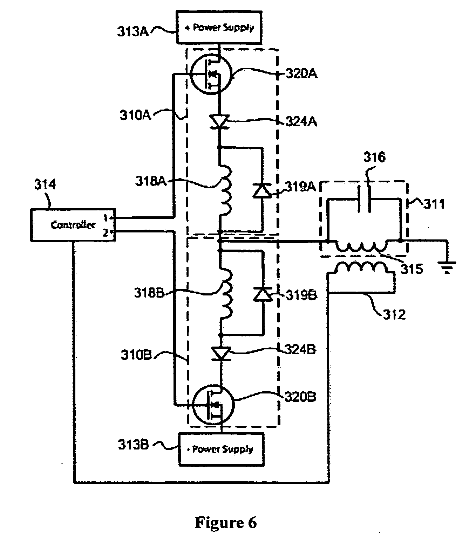

Figure 6 shows a configuration in which one end of the resonant circuit 311 is connected to zero volts, and the other end is driven symmetrically by being pulled towards either a positive supply 313A when switch 320A turns on, or a negative supply 313B when switch 320B turns on. The switch 320A connected to the positive supply 313A can be closed and opened at any time from when the voltage in the resonant circuit 311 is zero to the negative peak voltage in the resonant circuit 311. The switch must opened after it is closed within the same area. The switch 320B connected to the negative supply 313B can be closed and opened at any time from when the voltage in the resonant circuit 311 is zero to the positive peak voltage in the resonant circuit 311. The switch must opened after it is closed within the same area. Preferably, if phase is taken to equal zero when the resonant circuit voltage crosses zero (when the voltage swings from positive to negative), then switch 320A should be closed when the phase is between 0° and 10° and opened when the phase is between 80° and 90°. Similarly, switch 320B should be closed when the phase is between 180° and 190° and opened when the phase is between 260° and 270°. Preferably, the closing and opening of switch 320B occurs at a phase of around 180° to the respective closing and opening of switch 320A.

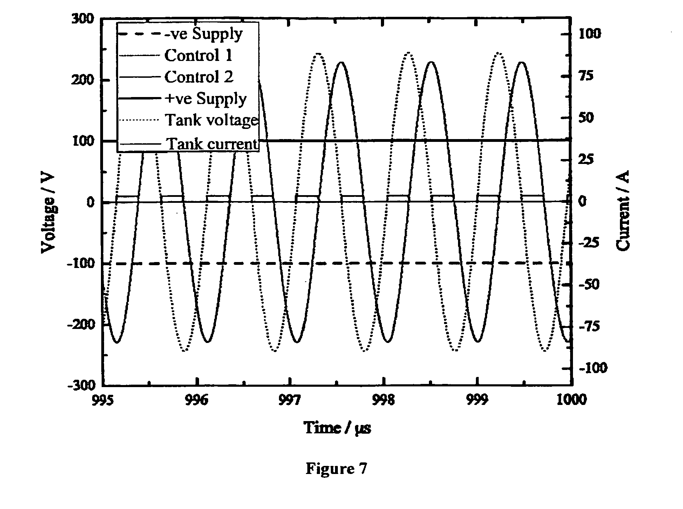

Typical waveforms are shown in Figure 7; for a power supply of +/- 100 V, the resonant circuit 311 reaches a peak-peak voltage swing of 487 V and peak current of 84 A. The current in the switches 320A and 320B is 15 A. This configuration achieves higher current in the resonant circuit 311 at the expense of greater complexity.

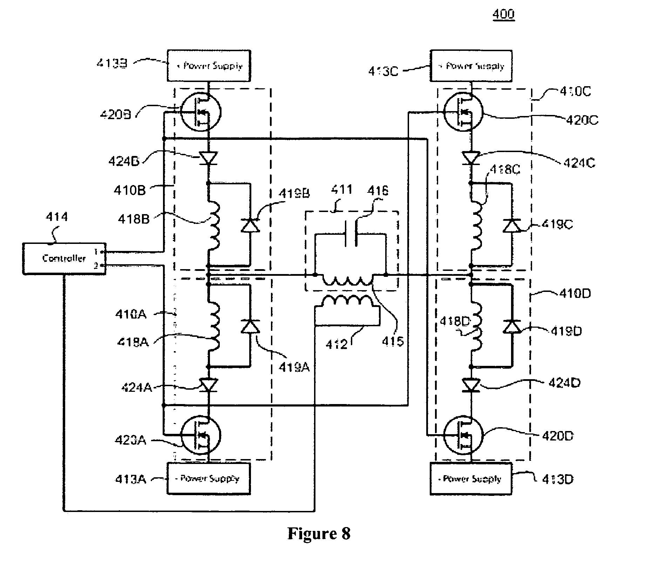

Figure 8 shows a configuration in which the two ends of the resonant circuit 411 are driven in anti-phase. Switch 420A turns on at the same time as switch 420C; switch 420B turns on at the same time as switch 420D. The switches 420B and 420D, connected to positive supply 413B and negative supply 413D respectively, can be closed and opened at any time from when the voltage in the resonant circuit 411 is zero to the negative peak voltage in the resonant circuit 411. The switch must opened after it is closed within the same area. The switches 420A and 420C, connected to the negative supply 413A and positive supply 413C respectively, can be closed and opened at any time from when the voltage in the resonant circuit 411 is zero to the positive peak voltage in the resonant circuit 411. The switch must opened after it is closed within the same area. Preferably, switches 420B and 420D are closed and opened at substantially the same time and switches 420A and 420C are closed and opened at substantially the same time. Preferably, if phase is taken to equal zero when the resonant circuit voltage crosses zero (when the voltage swings from positive to negative), then switches 420B and 420D should be closed when the phase is between 0° and 10° and opened when the phase is between 80° and 90°. Similarly, switches 420A and 420C should be closed when the phase is between 180° and 190° and opened when the phase is between 260° and 270°. It is also preferable that the closing and opening of switches 420A and 420C occur at a phase of around 180° to the respective closing and opening of switches 420B and 420D.

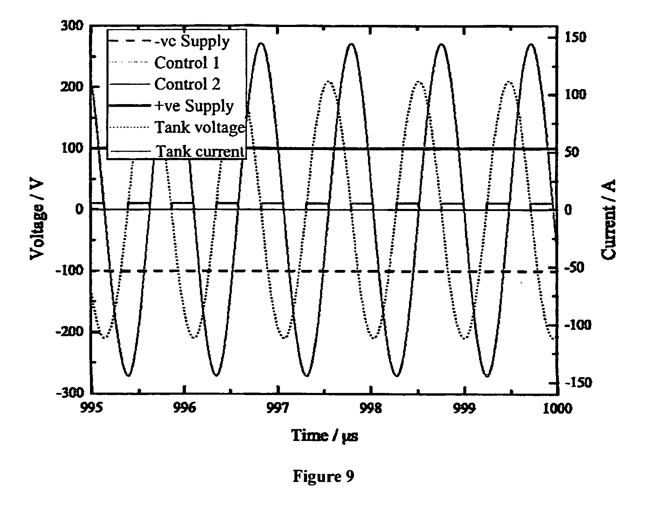

Typical waveforms are shown in Figure 9; for a power supply of +/- 100 V, the resonant circuit 411 reaches an effective peak-peak voltage swing of 840 V and peak current of 144 A. The current in the switches 420A to 420D is 17 A. This configuration achieves higher current in the resonant circuit 411 at the expense of greater complexity.

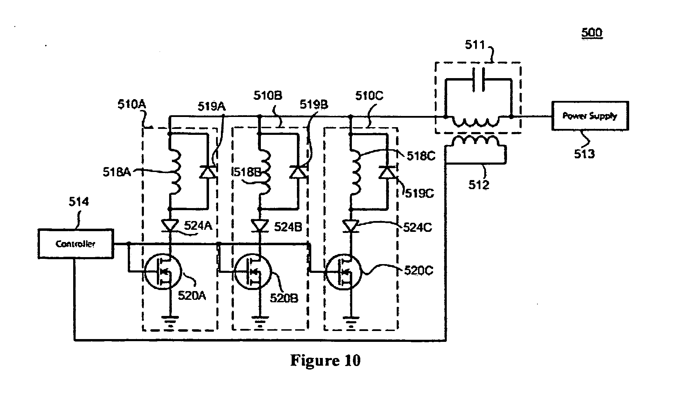

Figure 10 shows a configuration in which drive sections 510A, 510B, 510C are connected in parallel to share the load current. This reduces heat dissipation and stress on the drive components, which may in turn allow higher power operation to be achieved. Connecting the drive sections in parallel 510A, 510B, 510C, rather than just the switches 520A, 520B, 520C, is preferred because it enforces good current sharing and it reduces stress on the diodes. The load-sharing method shown in Figure 10 can also be applied in the half-bridge and full-bridge configurations of Figures 6 and 8.

The half-bridge, full-bridge and parallel configurations described above are exemplary. It is possible to have different numbers of controller and drive circuit configurations to drive a resonant circuit. For example, the full-bridge configuration described in figure 8 may employ a single controller which supplies the control signals to the drive circuits or at least one controller for each drive circuit may be employed. Also for example, the number of drive circuits shown in figure 10 can be increased or decreased.

An exemplary system 100 is designed so that it is possible to separate the modules (e.g. electronic circuit 10, resonant circuit 11, sensor 12, controller 14, etc...) of the system 100 so that the modules can be independently created, interchanged and used in different systems.

The modular design of the system 100 allows the user to change the resonant circuit 11 without having to retune or adjust the electronic circuit 10 or sensor 12. The user is able to change the configuration of the coil 15 and the capacitor 16 of resonant circuit 11. The magnetic field strength generated by the coil 15 is dependent upon the current flowing through the coil 15 and on its geometry and material. The user may change these properties to provide an appropriate magnetic field for his chosen application. The system 100 is capable of providing an alternating magnetic field with a magnetic field strength up to 80 kA/m (1000 Oe). The frequency of the alternating magnetic field may be in the range of from 100 kHz to 2 MHz, or even in excess of 2 MHz.

The type of sensor 12 may be chosen so that it operates at an optimised sensitivity for the relevant chosen frequency and magnetic field strength. The positioning of the sensor 12 may also optimise the feedback signal to the controller 14. For example, a sense coil 17 may be positioned at the end of coil 15 so that the common-mode capacitive coupling effect into the sense coil 17 is reduced.

Figure 12 shows an example of a coil connected to mounting plates to provide electrical contact, ensuring that the capacitor and coil are mounted as close as possible and also to increase the fluid cooling efficiency of an attached capacitor. The coil, capacitor and mounting plates can be housed in a hand-held shell to allow a user to manually position the coil in a desired area. The hand-held shell provides electrical isolation so the user can safely handle and position the coil. The hand-held structure may be connected to a control box, which comprises the electronic circuit. The hand-held structure may be connected to the control box via an umbilical which contains the electrical feed. The control box may further comprise a fluid cooling system which provides cooling to any of the components in the electronic circuit. The cooling system may also provide cooling feeds via the umbilical to the hand-held structure. The capacitor connected to the coil to form the resonant circuit may be placed within the hand-held structure or in the control box.

Optionally, a hand- held structure comprising the resonant circuit and the electronic circuit may be connected to an external power supply. A cooling system providing cooling fluid feeds may also be connected to the hand- held device. Alternatively, the hand- held device may comprise the cooling means (e.g. air- cooling fans) . In further variants, the power supply can be incorporated in the hand- held structure, or can be housed in the control box, for example via integrated power factor correction (PFC) units.

## Claims

1. An apparatus for driving a resonant circuit (11), the apparatus comprising:

    a first drive circuit (10), the first drive circuit (10) arranged to provide a drive current to said resonant circuit (11); and
a controller (14) coupled to said first drive circuit (10),
the first drive circuit (10) further comprising:

a first input adapted to receive a current from a power supply (13);
a switch (20);
a second input adapted to receive from said controller (14) a signal to control said switch (20); and
an output coupled to said resonant circuit (11);

wherein the controller (14) is adapted to receive from a sensor (12) a signal derived from the current flowing in said resonant circuit (11), wherein said sensor (12) is coupled to said controller (14), and
said controller (14) is configured to close said switch (20) to enable the drive current to flow through said resonant circuit (11) when said signal derived from said sensor (12) satisfies a first condition and said controller (14) is further configured to open said switch (20) to cause the drive current to stop flowing through the resonant circuit (11) when said signal derived from said sensor (12) satisfies a second condition, **characterized by** the first drive circuit (10) further comprising:

a first inductor (18), which acts to set the drive current through said resonant circuit (11) when the switch (20) is closed; and
a first diode (19) coupled across said first inductor (18), said first diode (19) arranged to enable current to continue to flow in the first inductor (18) when the switch (20) is open.

2. The apparatus according to claim 1, wherein said first inductor (18) enables peak current flowing in said resonant circuit (11) to substantially exceed the mean current provided at the first input,
and/or
wherein said controller (14) is further configured to control said switch (20) so as to cause current in said resonant circuit (11) to oscillate at a frequency substantially equal to the resonant frequency of the resonant circuit (11).

3. The apparatus according claim 1 or 2, wherein said first condition and said second condition are that the phase of the current, determined from said signal derived from said sensor (12), reaches first and second respective phase values,
and/or
wherein said drive circuit further comprises a second diode (24) coupled to said switch (20) to substantially stop current flow through said switch (20) in the reverse direction,
and/or
wherein said drive circuit (10) further comprises a second inductor (25) coupled to said resonant circuit (11) to provide smoothing of the current flowing through said resonant circuit (11),
and/or
wherein said first inductor (18) is configured to avoid excessive current surges in said switch (20).

4. The apparatus according to claim 3, wherein, if phase equals zero when the voltage in the resonant circuit (11) is at its positive peak, the first phase value is between 0° and 90° and the second phase value is between 90° and 270°.

5. The apparatus according to any one of claims 1 to 4, further comprising at least one additional drive circuit (310A, 310B; 410A, 410B, 410C, 410D; 510A, 510B, 510C),
wherein the second input of each additional drive circuit is coupled to said controller (314; 414; 514) and the output of each additional drive circuit is coupled to the resonant circuit (311; 411; 511).

6. An apparatus for driving a resonant circuit, the apparatus comprising:

a first and second apparatus (310A, 310B, 314) according to any one of claims 1-3,
wherein the polarity of the voltage applied at the first input of the first apparatus (310A) is opposite to the polarity of the voltage applied to the first input of the second apparatus (310B), and preferably wherein a positive voltage is applied to the first input of the first apparatus (310A) and a negative voltage is applied to the first input of the second apparatus (310B) and, if phase equals zero when the voltage in the resonant circuit (311) crosses zero from positive to negative, the first and second phase values of the first apparatus are between 0° and 90° wherein the second phase value is greater than the first phase value, and the first and second phase values of the second apparatus are between 180° and 270° wherein the second phase value is greater than the first phase value,
and/or
wherein said first and second conditions of said first apparatus are substantially at a phase of 180 degrees relative to the first and second conditions respectively of the second apparatus, and
preferably wherein the current flow through the resonant circuit (311) is substantially in one direction when the switch (320A) in the first apparatus is closed and substantially in the opposite direction when the switch (320B) in the second apparatus is closed.

7.  The apparatus according to any one of claims 1-3, further comprising one additional drive circuit (31 0B),

    wherein the second input of the additional drive circuit is coupled to said controller (314) and the output of the additional drive circuit is coupled to the resonant circuit (311).

8.  An apparatus for driving a resonant circuit, the apparatus comprising:

    a plurality of apparatuses according to any one of claims 1 to 4 or a plurality of apparatuses according to claim 7.

9.  The apparatus according to claim 7, wherein the polarity of the voltage applied at the first input of the first drive circuit (310A) is opposite to the polarity of the voltage applied at the first input of the additional drive circuit (310B), and

    preferably wherein a positive voltage is applied to the first input of the first drive circuit (310A) and a negative voltage is applied to the first input of the additional drive circuit (310B) and, if phase equals zero when the voltage in the resonant circuit crosses zero from positive to negative, the first and second phase values of the first drive circuit are between 0° and 90° wherein the second phase value is greater than the first phase value, and the first and second phase values of the additional drive circuit are between 180° and 270° wherein the second phase value is greater than the first phase value, and/or

    wherein the first and second conditions of the first drive circuit (310A) are substantially at a phase of 180 degrees relative to the first and second conditions respectively of the additional drive circuit (310B).

10. An apparatus for driving a resonant circuit comprising:

    a first and second apparatus according to claim 9, wherein the first drive circuit (410B) of the first apparatus is connected to one end of the resonant circuit (411) and the additional drive circuit (410D) of the first apparatus is connected to the opposite end of the resonant circuit (411), and the first drive circuit (410C) of the second apparatus is connected to said opposite end of the resonant circuit (411) and the additional drive circuit (410A) of the second apparatus is connected to said one end of the resonant circuit (411), and

preferably wherein a positive voltage is applied to the first input of the first drive circuits (410B, 410C) of the first and second apparatuses and a negative voltage is applied to the first input of the additional drive circuits (410D, 410A) of the first and second apparatuses and, if phase equals zero when the voltage in the resonant circuit crosses zero from positive to negative, the first and second phase values of the first drive circuit and additional drive circuit of the first apparatus are between 0° and 90° wherein the second phase value is greater than the first phase value, and the first and second phase values of the first drive circuit and additional drive circuit of the second apparatus are between 180° and 270° wherein the second phase value is greater than the first phase value, and/or

wherein said first and second conditions of the first and additional drive circuit of the first apparatus are substantially the same and first and second conditions of the first and additional drive circuit of the second apparatus are substantially the same and the first and second conditions of said first apparatus are substantially at a phase of 180 degrees relative to the first and second conditions respectively of the second apparatus, and

preferably wherein the current flow through the resonant circuit (411) is in one direction when the switches (420B, 420D) in the first apparatus are closed and in the opposite direction when the switches (420C, 420A) in the second apparatus are closed.

11. The apparatus according to any one of claims 1-3, further comprising a second, third and fourth drive circuit (410B, 410C, 410D), wherein:

    the second input of the first, second, third and fourth drive circuits are coupled to said controller (414);
    the polarity of the voltage applied at the first inputs of the first and fourth drive circuits (410A, 410D) is opposite to the polarity of the voltage applied at the first inputs of the second and third drive circuits (410B, 410C); and
    the outputs of the first and second drive circuits (410A, 410B) are connected to one end of the resonant circuit (411) and the outputs of the third and fourth drive circuits (410C, 410D) are connected to the opposite end of the resonant circuit (411),

    preferably wherein a positive voltage is applied to the first input of the second and third drive circuits (410B, 410C) and a negative voltage is applied to the first input of the first and fourth drive circuits (410A, 410D) and, if phase equals zero when the voltage in the resonant circuit crosses zero from positive to negative, the first and second phase values of the second and fourth drive circuits are between 0° and 90° wherein the second phase value is greater than the first phase value, and the first and second phase values of the first and third drive circuits are between 180° and 270° wherein the second phase value is greater than the first phase value,

and/or

wherein the first and second conditions of the first and third drive circuits (410A, 410C) are substantially the same and first and second conditions of the second and fourth drive circuits (410B, 410D) are substantially the same and the first and second conditions of the first and third drive circuits (410A, 410C) are substantially at a phase of 180 degrees relative to the first and second conditions respectively of the second and fourth drive circuits (410B, 410D) and preferably wherein the current flow through the resonant circuit (411) is in one direction when the switches (420A, 420C) in the first and third drive circuits (410A, 410C) are closed and in the opposite direction when the switches (420B, 420D) in the second and fourth drive circuits (410B, 410D) are closed.

**12.** The apparatus according to any one of claims 4 to 11, wherein at least two drive circuits (510A, 510B, 510C) are connected in parallel.

**13.** A system comprising:

an apparatus (10, 14) according to any one of the preceding claims;
a resonant circuit (11) comprising a magnetising coil (15) connected to said apparatus; and
a sensor (12) adapted to derive a signal related to the current flowing in said resonant circuit (11).

**14.** The system according to claim 13, wherein said magnetising coil (15) is interchangeably connectable to said apparatus,
and/or
wherein said sensor (12) is a sense coil (17) inductively coupled to said magnetising coil (15) to induce in said sense coil (17) a signal related to the current in said magnetising coil (15),
and/or
wherein said magnetising coil (15) is adapted to generate an alternating magnetic field with a peak magnetic field strength in the range of from 0 to 16 kA/m (200 Oe),
and/or
wherein said magnetising coil (15) is adapted to generate an alternating magnetic field with a frequency in the range of from 100kHz to 2 MHz,
and/or
further comprising at least one power supply (13) adapted to provide power to said apparatus, wherein the power supply (13) is adapted to supply a voltage in the range of from 0 to 150 V and a mean current in the range from 0 to 2 A,
and/or
wherein the magnetising coil (15) or the resonant circuit (11) is comprised within a hand held device,
and/or
further comprising a fluid cooling system for cooling at least one component of said system.

**15.** A magnetic field hyperthermia device comprising an apparatus or system according to any one of the preceding claims.

**Patentansprüche**

**1.** Vorrichtung zum Antreiben eines Schwingkreises (11), wobei die Vorrichtung umfasst:

einen ersten Antriebskreis (10), wobei der erste Antriebskreis (10) angeordnet ist, um dem Schwingkreis (11) einen Antriebsstrom bereitzustellen; und
eine Steuerung (14), die mit ersten Antriebskreis (10) gekoppelt ist, wobei der erste Antriebskreis (10) weiterhin umfasst:

einen ersten, für den Empfang eines Stroms von einer Energiequelle (13) ausgelegten Eingang,
einen Schalter (20);
einen zweiten, für den Empfang eines Signals von der Steuerung (14) zur Steuerung des Schalters (20) ausgelegten Eingang; und
einen mit dem Schwingkreis (11) gekoppelten Ausgang;

wobei die Steuerung (14) eingerichtet ist, von einem Sensor (12) ein von dem in dem Schwingkreis (11) fließenden Strom abgeleitetes Signal zu empfangen, wobei der Sensor (12) mit der Steuerung (14) gekoppelt ist, und
wobei die Steuerung (14) konfiguriert ist, den Schalter (20) zu schließen, um dem Antriebsstrom zu ermöglichen, durch den Schwingkreis (11) zu fließen, wenn das von dem Sensor (12) abgeleitete Signal eine erste Bedingung erfüllt, und wobei die Steuerung (14) weiterhin konfiguriert ist, den Schalter (20) zu öffnen, wodurch der Antriebsstrom aufhört durch den Schwingkreis (11) zu fließen, wenn das von dem Sensor (12) abgeleitete Signal eine zweite Bedingung erfüllt,

**dadurch gekennzeichnet, dass** der erste Antriebskreis (10) weiterhin umfasst:

einen ersten Induktor (18), der zum Einstellen des Antriebsstroms durch den Schwingkreis (11) wirkt, wenn der Schalter (20) geschlossen ist; und
eine erste über den ersten Induktor (18) gekoppelte Diode (19), wobei die erste Diode (19) so angeordnet ist, dass Strom weiterhin im ersten Induktor (18) fließen kann, wenn der Schalter (20) offen ist.

2. Vorrichtung nach Anspruch 1, wobei der erste Induktor (18) den Fluss eines Spitzenstroms in dem Schwingkreis (11) ermöglicht, der wesentlich höher ist als der am ersten Eingang zur Verfügung gestellte mittlere Strom,
und/oder
wobei die Steuerung (14) ferner konfiguriert ist, den Schalter (20) so zu steuern, dass der in dem Schwingkreis (11) fließende Strom mit einer Frequenz oszilliert, die im Wesentlichen gleich der Resonanzfrequenz des Schwingkreises (11) ist.

3. Vorrichtung nach Anspruch 1 oder 2, wobei die erste Bedingung und die zweite Bedingung sind, dass die Phase des Stroms, die durch das von dem Sensor (12) abgeleitete Signal bestimmt wird, jeweils einen ersten und zweiten Phasenwert erreicht,
und/oder
wobei der Antriebkreis ferner eine zweite, an den Schalter (20) gekoppelte Diode (24) umfasst, um einen Stromfluss durch den Schalter (20) in der umgekehrten Richtung im Wesentlichen zu verhindern,
und/oder
wobei der Antriebskreis (10) weiterhin einen zweiten, mit dem Schwingkreis (11) gekoppelten Induktor (25) zum Glätten des Stroms, der durch den Schwingkreis (11) fließt, umfasst,
und/oder
wobei der erste Induktor (18) konfiguriert ist, übermäßige Stromspitzen in dem Schalter (20) zu vermeiden.

4. Vorrichtung nach Anspruch 3, wobei der erste Phasenwert zwischen 0° und 90° liegt und der zweite Phasenwert zwischen 90° und 270° liegt, falls Phase gleich Null ist, wenn die Spannung in dem Schwingkreis (11) ihren positiven Spitzenwert erreicht.

5. Vorrichtung nach einem der Ansprüche 1 bis 4, die weiterhin mindestens einen zusätzlichen Antriebskreis (310A, 310B; 410A, 410B, 410C, 410D; 510A, 510B, 510C) umfasst,
wobei der zweite Eingang jedes zusätzlichen Antriebskreises mit der Steuerung (314; 414; 514) gekoppelt ist und der Ausgang jedes zusätzlichen Antriebskreises mit dem Schwingkreis (311; 411; 511) gekoppelt ist.

6. Vorrichtung zum Antreiben eines Schwingkreises, wobei die Vorrichtung umfasst:

eine erste und zweite Vorrichtung (310A, 310B, 314) nach einem der Ansprüche 1-3,
wobei die Polarität der am ersten Eingang der ersten Vorrichtung (310A) angelegten Spannung entgegengesetzt zu der Polarität der am ersten Eingang der zweiten Vorrichtung (310B) angelegten Spannung ist, und
wobei vorzugsweise eine positive Spannung an den ersten Eingang der ersten Vorrichtung (310A) und eine negative Spannung an den ersten Eingang der zweiten Vorrichtung (310B) angelegt sind und wobei, falls Phase gleich Null ist, wenn die Spannung in dem Schwingkreis (311) Null von positiv nach negativ passiert, der erste und zweite Phasenwert der ersten Vorrichtung zwischen 0° und 90° liegen, wobei der zweite Phasenwert größer als der erste Phasenwert ist, und der erste und zweite Phasenwert der zweiten Vorrichtung zwischen 180° und 270° liegen, wobei der zweite Phasenwert größer als der erste Phasenwert ist,
und/oder
wobei die erste und die zweite Bedingung der ersten Vorrichtung im Wesentlichen bei einer Phase von 180 Grad relativ zur ersten und zweiten Bedingung der zweiten Vorrichtung sind, und
wobei vorzugsweise der Strom im Wesentlichen in einer Richtung durch den Schwingkreis (311) fließt, wenn der Schalter in der ersten Vorrichtung geschlossen ist, und im Wesentlichen in die entgegengesetzte Richtung fließt, wenn der Schalter (320B) in der zweiten Vorrichtung geschlossen ist.

7. Vorrichtung nach einem der Ansprüche 1 -3, die ferner einen zusätzlichen Antriebskreis (310B) umfasst,
wobei der zweite Eingang des zusätzlichen Antriebskreises an die Steuerung (314) gekoppelt ist, und der Ausgang des zusätzlichen Antriebskreises an den Schwingkreis (311) gekoppelt ist.

8. Vorrichtung zum Antreiben eines Schwingkreises, wobei die Vorrichtung umfasst:

eine Vielzahl von Vorrichtungen nach einem der Ansprüche 1-4 oder eine Vielzahl von Vorrichtungen nach Anspruch 7.

9. Vorrichtung nach Anspruch 7, wobei die Polarität der am ersten Eingang des ersten Antriebskreises (310A) angelegten Spannung entgegengesetzt zur Polarität der am ersten Eingang des zusätzlichen Antriebskreises (310B) angelegten Spannung ist, und
wobei vorzugsweise eine positive Spannung an dem ersten Eingang des ersten Antriebskreises (310A) angelegt ist und eine negative Spannung an den ersten Eingang des zusätzlichen Antriebskreises (310B) angelegt ist, und wobei, falls Phase gleich Null ist, wenn die Spannung in dem Schwingkreis Null von positiv nach negativ passiert, der erste und zweite Phasenwert des ersten Antriebskreises zwischen 0° und 90° liegen, wobei der zweite Phasenwert größer ist als der erste Phasenwert, und der erste und zweite Phasenwert des zusätzlichen Antriebskreises zwischen 180° und 270° liegen, wobei der zweite Phasenwert größer ist als der erste Phasenwert,
und/oder
wobei die erste und die zweite Bedingung des ersten Antriebskreises (310A) im Wesentlichen bei einer Phase von 180 Grad relativ zur ersten und zweiten Bedingung des zusätzlichen Antriebskreises sind.

10. Vorrichtung zum Antreiben eines Schwingkreises, die umfasst:

eine erste und zweite Vorrichtung nach Anspruch 9, wobei der erste Antriebskreis (410B) der ersten Vorrichtung mit einem Ende des Schwingkreises (411) verbunden ist und der zusätzliche Antriebskreis (410D) der ersten Vorrichtung mit dem entgegengesetzten Ende des Schwingkreises (411) verbunden ist, und wobei der erste Antriebskreis (410C) der zweiten Vorrichtung mit dem gegenüberliegenden Ende des Schwingkreises (411) verbunden ist und der zusätzliche Antriebskreis (410A) der zweiten Vorrichtung mit dem einen Ende des Schwingkreises (411) verbunden ist, und
wobei vorzugsweise eine positive Spannung an dem ersten Eingang der ersten Antriebskreise (410B, 410C) der ersten und zweiten Vorrichtung und eine negative Spannung an den ersten Eingang der zusätzlichen Antriebskreise (410D, 410A) der ersten und zweiten Vorrichtung angelegt wird und wobei, falls Phase gleich Null ist, wenn die Spannung in dem Schwingkreis Null von positiv nach negativ passiert, der erste und zweite Phasenwert des ersten Antriebskreises und des zusätzlichen Antriebskreises der ersten Vorrichtung zwischen 0° und 90° liegen, wobei der zweite Phasenwert größer ist als der erste Phasenwert, und wobei der erste und zweite Phasenwert des ersten Antriebskreises und des zusätzlichen Antriebskreises der zweiten Vorrichtung zwischen 180° und 270° liegen, wobei der zweite Phasenwert größer ist als der erste Phasenwert
und/oder
wobei die erste und die zweite Bedingung des ersten und zusätzlichen Antriebskreises der ersten Vorrichtung im Wesentlichen gleich sind und die erste und die zweite Bedingung des ersten und des zusätzlichen Antriebskreises der zweiten Vorrichtung im Wesentlichen gleich sind und die erste und die zweite Bedingung der ersten Vorrichtung im Wesentlichen bei einer Phase von 180 Grad relativ zu jeweils der ersten und zweiten Bedingung der zweiten Vorrichtung sind, und
wobei vorzugsweise der Strom in einer Richtung durch den Schwingkreis (411) fließt, wenn die Schalter (420B, 420D) in der ersten Vorrichtung geschlossen sind und in der entgegengesetzten Richtung, wenn die Schalter (420C, 420A) in der zweiten Vorrichtung geschlossen sind.

11. Vorrichtung nach einem der Ansprüche 1-3, die ferner einen zweiten, dritten und vierten Antriebskreis (410B, 410C, 410D) umfasst, wobei:

der zweite Eingang des ersten, zweiten, dritten und vierten Antriebskreises mit der Steuerung (414) gekoppelt sind;
wobei die Polarität der an den ersten Eingängen des ersten und vierten Antriebskreises (410A, 410D) angelegten Spannung entgegengesetzt zu der Polarität der an den ersten Eingängen der zweiten und dritten Antriebskreise (410B, 410C) angelegten Spannung ist; und
wobei die Ausgänge des ersten und zweiten Antriebskreises (410A, 410B) mit einem Ende des Schwingkreises (411) verbunden sind und die Ausgänge des dritten und vierten Antriebskreises (410C, 410D) mit dem gegenüberliegenden Ende des Schwingkreises (411) verbunden sind,
wobei vorzugsweise eine positive Spannung an den ersten Eingang des zweiten und dritten Antriebskreises (410B, 410C) angelegt ist und eine negative Spannung an den ersten Eingang des ersten und vierten Antriebskreises (410A, 410D) angelegt ist, und wobei, falls Phase gleich Null ist, wenn die Spannung in dem Schwingkreis Null von positiv nach negativ passiert, der erste und zweite Phasenwert des zweiten und vierten Antriebskreises zwischen 0° und 90° liegen, wobei der zweite Phasenwert größer ist als der erste Phasenwert, und der erste und zweite Phasenwert des ersten und dritten Antriebskreises zwischen 180° und 270° liegen, wobei der zweite Phasenwert größer ist als der erste Phasenwert

und/oder

wobei die erste und die zweite Bedingung des ersten und dritten Antriebskreises (410A, 410C) im Wesentlichen gleich sind und die erste und die zweite Bedingung des zweiten und vierten Antriebskreises (410B, 410D) im Wesentlichen gleich sind und

wobei die erste und die zweite Bedingung der ersten und dritten Antriebskreise (410A, 410C) im Wesentlichen bei einer Phase von 180 Grad relativ zu jeweils der ersten und zweiten Bedingung der zweiten und vierten Antriebskreise (410B, 410D) sind, und

wobei vorzugsweise der Strom in einer Richtung durch den Schwingkreis (411) fließt, wenn die Schalter (420A, 420C) in dem ersten und dritten Antriebskreis (410A, 410C) geschlossen sind und in der entgegengesetzten Richtung, wenn die Schalter (420B, 420D) in dem zweiten und vierten Antriebskreis (410B, 410D) geschlossen sind.

12. Vorrichtung nach einem der Ansprüche 4 bis 11, wobei mindestens zwei Antriebskreise (510A, 510B, 510C) parallel geschaltet sind.

13. System, umfassend:

eine Vorrichtung (10, 14) nach einem der voranstehenden Ansprüche;
einen Schwingkreis (11) mit einer Magnetspule (15), die mit der Vorrichtung verbunden ist; und
einen Sensor (12), der eingerichtet ist, ein Signal in Abhängigkeit des Stromflusses in dem Schwingkreis (11) abzuleiten.

14. System nach Anspruch 13, wobei die Magnetspule (15) auswechselbar mit der Vorrichtung verbunden werden kann,
und/oder
wobei der Sensor (12) eine induktiv mit der Magnetspule (15) gekoppelte Sensorspule (17) zum Induzieren eines Signals in der Sensorspule in Abhängigkeit von dem Strom in der Magnetspule (15) ist
und/oder
wobei die Magnetspule (15) eingerichtet ist, ein magnetisches Wechselfeld mit einem Spitzenwert der magnetischen Feldstärke im Bereich von 0 bis 16 kA/m (200 Oe) zu erzeugen,
und/oder
wobei die Magnetspule (15) eingerichtet ist, ein magnetisches Wechselfeld mit einer Frequenz im Bereich von 100 kHz bis 2 MHz zu erzeugen,
und/oder
ferner umfassend mindestens eine Energiequelle (13) zur Energieversorgung der Vorrichtung, wobei die Energieversorgung (13) zur Versorgung einer Spannung im Bereich von 0 bis 150 V und eines mittleren Stroms im Bereich von 0 bis 2 A ausgelegt ist,
und/oder
wobei die Magnetspule (15) oder der Schwingkreis (11) in einem Handgerät enthalten sind,
und/oder
ferner umfassend ein Fluid-Kühlsystem zum Kühlen mindestens einer Komponente des Systems.

15. Magnetfeld-Hyperthermievorrichtung umfassend eine Vorrichtung oder ein System nach einem der vorhergehenden Ansprüche.

**Revendications**

1. Appareil, destiné à piloter un circuit résonnant (11), l'appareil comprenant :

un premier circuit (10) de pilotage, le premier circuit (10) de pilotage étant agencé pour fournir un courant d'attaque audit circuit résonnant (11) et
un régulateur (14), couplé audit premier circuit (10) de pilotage ;
le premier circuit (10) de pilotage comprenant, en outre :

une première entrée, adaptée pour recevoir un courant d'une alimentation (13) en courant ;
un commutateur (20) ;
une seconde entrée, adaptée pour recevoir dudit régulateur (14) un signal pour régler ledit commutateur (20) et
une sortie, couplée audit circuit résonnant (11) ;

dans lequel le régulateur (14) est adapté pour recevoir d'un détecteur (12) un signal, issu du courant qui circule dans ledit circuit résonnant (11), dans lequel ledit détecteur (12) est couplé audit régulateur (14) et
ledit régulateur (14) est configuré pour fermer ledit commutateur (20), pour permettre au courant d'attaque de circuler dans ledit circuit résonnant (11), lorsque ledit signal, issu dudit détecteur (12), satisfait une première condition et ledit régulateur (14) est configuré, en outre, pour ouvrir ledit commutateur (20), pour amener le courant d'attaque à cesser de circuler dans le circuit résonnant (11), lorsque ledit signal, issu dudit détecteur (12), satisfait une seconde condition,
**caractérisé par** le premier circuit (10) de pilotage qui comprend, en outre :

une première bobine (18) d'induction, qui agit pour régler le courant d'attaque dans ledit circuit résonnant (11), lorsque le commutateur (20) est fermé et

une première diode (19), couplée à l'ensemble de ladite première bobine (18) d'induction, ladite première diode (19) étant agencée pour permettre au courant de continuer à circuler dans la première bobine (18) d'induction, lorsque le commutateur (20) est ouvert.

2. Appareil selon la revendication 1, dans lequel ladite première bobine (18) d'induction permet au courant de crête, qui circule dans ledit circuit résonnant (11), de dépasser sensiblement le courant moyen, fourni à la première entrée

et / ou

dans lequel ledit régulateur (14) est configuré, en outre, pour régler ledit commutateur (20), de façon à amener le courant dans ledit circuit résonnant (11) à osciller à une fréquence sensiblement égale à la fréquence de résonance du circuit résonnant (11).

3. Appareil selon la revendication 1 ou 2, dans lequel ladite première condition et ladite seconde condition sont que la phase du courant, déterminée à partir dudit signal, issu dudit détecteur (12), atteint les première et seconde valeurs respectives de phase

et / ou

dans lequel ledit circuit de pilotage comprend, en outre, une seconde diode (24), couplée audit commutateur (20), pour empêcher sensiblement le courant de circuler dans ledit commutateur (20) en sens inverse

et / ou

dans lequel ledit circuit (10) de pilotage comprend une seconde bobine (25) d'induction, couplée audit circuit résonnant (11), pour fournir le lissage du courant qui circule dans ledit circuit résonnant (11)

et / ou

dans lequel ladite première bobine (18) d'induction est configurée pour éviter qu'une surcharge de courant ne monte subitement dans ledit commutateur (20).

4. Appareil selon la revendication 3, dans lequel, si la phase est égale à zéro, lorsque la tension dans le circuit résonnant (11) est à sa crête positive, la première valeur de phase est entre 0° et 90° et la seconde valeur de phase est entre 90° et 270°.

5. Appareil selon l'une quelconque des revendications 1 à 4, comprenant, en outre, au moins un circuit (310A, 310B ; 410A, 410B, 410C, 410D ; 510A, 510B, 510C) supplémentaire de pilotage,

dans lequel la seconde entrée de chaque circuit supplémentaire de pilotage est couplée audit régulateur (314 ; 414 ; 514) et la sortie de chaque circuit supplémentaire de pilotage est couplée au circuit résonnant (311 ; 411 ; 511).

6. Appareil, destiné à piloter un circuit résonnant, l'appareil comprenant :

un premier et un second appareil (310A, 310B, 314), selon l'une quelconque des revendications 1 à 3,

dans lequel la polarité de la tension, appliquée à la première entrée du premier appareil (310A), est opposée à la polarité de la tension, appliquée à la première entrée du second appareil (310B) et

dans lequel, de préférence, une tension positive est appliquée à la première entrée du premier appareil (310A) et une tension négative est appliquée à la première entrée du second appareil (310B) et, si la phase est égale à zéro, lorsque la tension dans le circuit résonnant (311) passe par zéro de positif à négatif, les première et seconde valeurs de phase du premier appareil sont entre 0° et 90°, dans lequel la seconde valeur de phase est supérieure à la première valeur de phase et les première et seconde valeurs de phase du second appareil sont entre 180° et 270°, dans lequel la seconde valeur de phase est supérieure à la première valeur de phase

et / ou

dans lequel lesdites première et seconde conditions dudit premier appareil sont sensiblement à une phase de 180 degrés par rapport aux première et seconde conditions, respectivement, du second appareil et

dans lequel, de préférence, le flux de courant dans le circuit résonnant (311) est sensiblement dans un sens, lorsque le commutateur (320A) dans le premier appareil est fermé et sensiblement dans le sens opposé, lorsque le commutateur (320B) dans le second appareil est fermé.

7. Appareil selon l'une quelconque des revendications 1 à 3, comprenant, en outre un circuit (310B) supplémentaire de pilotage,

dans lequel la seconde entrée du circuit supplémentaire de pilotage est couplée audit régulateur (314) et la sortie du circuit supplémentaire de pilotage est couplée au circuit résonnant (311).

8. Appareil, destiné à piloter un circuit résonnant, l'appareil comprenant :

une pluralité d'appareils, selon l'une quelconque des revendications 1 à 4 ou une pluralité d'appareils, selon la revendication 7.

9. Appareil selon la revendication 7, dans lequel la polarité de la tension, appliquée à la première entrée du premier circuit (310A) de pilotage est opposée à la polarité de la tension, appliquée à la première entrée du circuit (310B) supplémentaire de pilotage et dans lequel, de préférence, une tension positive est appliquée à la première entrée du premier circuit (310A) de pilotage et une tension négative est appliquée à la première entrée du circuit (310B) supplémentaire de pilotage et, si la phase est égale à zéro, lorsque la tension dans le circuit résonnant passe par zéro de positif à négatif, les première et seconde valeurs de phase du premier circuit de pilotage sont entre 0° et 90°, dans lequel la seconde valeur de phase est supérieure à la première valeur de phase et les première et seconde valeurs de phase du circuit supplémentaire de pilotage sont entre 180° et 270°, dans lequel la seconde valeur de phase est supérieure à la première valeur de phase

et / ou

dans lequel les première et seconde conditions du premier circuit (310A) de pilotage sont sensiblement à une phase de 180 degrés par rapport aux première et seconde conditions, respectivement, du circuit (310B) supplémentaire de pilotage.

10. Appareil, destiné à piloter un circuit résonnant, comprenant :

un premier et un second appareil, selon la revendication 9, dans lequel le premier circuit (410B) de pilotage du premier appareil est connecté à une extrémité du circuit résonnant (411) et le circuit (410D) supplémentaire de pilotage du premier appareil est connecté à l'extrémité opposée du circuit résonnant (411) et le premier circuit (410C) de pilotage du second appareil est connecté à ladite extrémité opposée du circuit résonnant (411) et le circuit (410A) supplémentaire de pilotage du second appareil est connecté à ladite extrémité du circuit résonnant (411) et dans lequel, de préférence, une tension positive est appliquée à la première entrée des premiers circuits (410B, 410C) de pilotage des premier et second appareils et une tension négative est appliquée à la première entrée des circuits (410D, 410A) supplémentaires de pilotage des premier et second appareils et, si la phase est égale à zéro, lorsque la tension dans le circuit résonnant passe par zéro de positif à négatif, les première et seconde valeurs de phase du premier circuit de pilotage et du circuit supplémentaire de pilotage du premier appareil sont entre 0° et 90°, dans lequel la seconde valeur de phase est supérieure à la première valeur de phase et les première et seconde valeurs de phase du premier circuit de pilotage et du circuit supplémentaire de pilotage du second appareil sont entre 180° et 270°, dans lequel la seconde valeur de phase est supérieure à la première valeur de phase

et / ou

dans lequel lesdites première et seconde conditions du premier circuit de pilotage et du circuit supplémentaire de pilotage du premier appareil sont sensiblement les mêmes et les première et seconde conditions du premier circuit de pilotage et du circuit supplémentaire de pilotage du second appareil sont sensiblement les même et les première et seconde conditions dudit premier appareil sont sensiblement à une phase de 180 degrés par rapport aux première et seconde conditions, respectivement, du second appareil et

dans lequel, de préférence, le flux de courant dans le circuit résonnant (411) est dans un sens, lorsque les commutateurs (420B, 420D) dans le premier appareil sont fermés et dans le sens opposé, lorsque les commutateurs (420C, 420A) dans le second appareil sont fermés.

11. Appareil selon l'une quelconque des revendications 1 à 3, comprenant, en outre un deuxième, troisième et quatrième circuit (410B, 410C, 410D) de pilotage, dans lequel :

la seconde entrée des premier, deuxième, troisième et quatrième circuits de pilotage est couplée audit régulateur (414) ;

la polarité de la tension, appliquée aux premières entrées des premier et quatrième circuits (410A, 410D) de pilotage est opposée à la polarité de la tension, appliquée aux premières entrées des deuxième et troisième circuits (410B, 410C) de pilotage et

les sorties des premier et deuxième circuits (410A, 410B) de pilotage sont connectées à une extrémité du circuit résonnant (411) et les sorties des troisième et quatrième circuits (410C, 410D) de pilotage sont connectées à l'extrémité opposée du circuit résonnant (411),

dans lequel, de préférence, une tension positive est appliquée à la première entrée des deuxième et troisième circuits (410B, 410C) de pilotage et une tension négative est appliquée à la première entrée des premier et quatrième circuits (410A, 410D) de pilotage et, si la phase est égale zéro, lorsque la tension dans le circuit résonnant passe par zéro de positif à négatif, les première et seconde valeurs de phase des deuxième et quatrième circuits de pilotage sont entre 0° et 90°, dans lequel la seconde valeur de phase est supérieure à la première valeur de phase et les première et seconde valeurs de phase des premier et troisième circuits de pilotage sont entre 180° et 270°, dans lequel la seconde valeur de phase est supérieure à la première valeur de phase

et / ou

dans lequel les première et seconde conditions des premier et troisième circuits (410A, 410C) de pilotage sont sensiblement les mêmes et les première et seconde conditions des deuxième et quatrième circuits (410B, 410D) de pilotage sont sensiblement les mêmes et

les première et seconde conditions des premier et troisième circuits (410A, 410C) de pilotage sont sensiblement à une phase de 180 degrés par rapport aux première et seconde conditions, respectivement, des deuxième et quatrième circuits (410B, 410D) de pilotage et

dans lequel, de préférence, le flux de courant dans le circuit résonnant (411) est dans un sens, lorsque les commutateurs (420A, 420C) dans les premier et troisième circuits (410A, 410C) de pilotage sont fermés et dans le sens opposé, lorsque les commutateurs (420B, 420D) dans les deuxième et quatrième circuits (410B, 410D) de pilotage sont fermés.

12. Appareil selon l'une quelconque des revendications 4 à 11, dans lequel au moins deux circuits (510A, 510B, 510C) de pilotage sont connectés en parallèle.

13. Système, comprenant :

un appareil (10, 14) selon l'une quelconque des revendications précédentes,
un circuit résonnant (11), comprenant une bobine (15) d'aimantation, connectée audit appareil
et
un détecteur (12), adapté pour dériver un signal, lié au courant qui circule dans ledit circuit résonnant (11).

14. Système selon la revendication 13, dans lequel ladite bobine (15) d'aimantation peut être connectée de manière interchangeable audit appareil

et / ou

dans lequel ledit détecteur (12) est une bobine (17) de mesure, couplée de manière inductive à ladite bobine (15) d'aimantation, pour induire, dans ladite bobine (17) de mesure, un signal, lié au courant dans ladite bobine (15) d'aimantation

et / ou

dans lequel ladite bobine (15) d'aimantation est adaptée pour produire un champ magnétique alternatif, avec une intensité maximale du champ magnétique dans la plage de 0 à 16 kA/m (200 Oe)

et / ou

dans lequel la bobine (15) d'aimantation est adaptée pour produire un champ magnétique alternatif, avec une fréquence dans la plage de 100 kHz à 2 MHz

et / ou

comprenant, en outre, au moins une alimentation (13) en courant, adaptée pour fournir du courant audit appareil, dans lequel l'alimentation (13) en courant est adaptée pour fournir une tension dans la plage de 0 à 150 V et un courant moyen dans la plage de 0 à 2 A

et / ou

dans lequel la bobine (15) d'aimantation ou le circuit résonnant (11) est compris(e) dans un dispositif portatif

et / ou

comprenant, en outre, un système de refroidissement à fluide, destiné à refroidir au moins un composant dudit système.

15. Dispositif d'hyperthermie à champ magnétique, comprenant un appareil ou un système, selon l'une quelconque des revendications précédentes.

**Figure 1**

Figure 2

**Figure 3**

**Figure 4**

Figure 5

**Figure 6**

Figure 7

**Figure 8**

Figure 9

**Figure 10**

Figure 11

Figure 12

**EP 2 389 786 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5236410 A
- DE 102005059751
- FR 2904873
- US 20050067410 A